# EUROPEAN PATENT APPLICATION

(11) **EP 4 840 862 A1**
(43) Date of publication of application: **18.11.2026**
(21) Application number: 25738385.1
(22) Date of filing: 07.01.2025
(51) Int. Cl.: C07D 491/22, A61K 31/4745, A61K 31/496, A61P 35/00

(54) **PREPARATION FOR AND USE OF 7-HYDROXYLAMINE-SUBSTITUTED ALKYL CAMPTOTHECIN DERIVATIVES**

(30) Priority: 08.01.2024 CN 202410024968
(71) Applicant: Hangzhou Adcoris Biopharma Co., Ltd, Hangzhou, Zhejiang 310052 (CN)
(72) Inventor: MIAO, Zhenwei, Hangzhou, Zhejiang 310052 (CN); HUANG, Yunsheng, Hangzhou, Zhejiang 310052 (CN)
(74) Representative: Elzaburu S.L.P.
(86) International application number: PCT/CN2025/070910
(87) International publication number: WO 2025/148854

(57) **Abstract**

The present invention relates to the field of medicines, and specifically provides a series of 7-hydroxylamine-substituted alkyl camptothecin derivatives, a preparation method therefor and a use thereof. The compound has a structure represented by formula (I), the definition of each group in the formula is detailed in the description, and the compound has good anti-cancer activity.

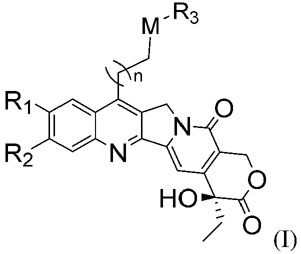

## Description

The present application claims priority to Chinese Patent Application No. 2024100249681, filed on January 8, 2024, which is incorporated herein by reference in its entirety.

### TECHNICAL FIELD

The present disclosure relates to the field of medicines. Specifically, the present disclosure provides a series of 7-hydroxylamine-substituted alkyl camptothecin derivatives, a preparation method thereof and use thereof.

### BACKGROUND

Camptothecin and derivatives thereof have inhibitory activity against topoisomerase I (Top1), especially have strong inhibitory activity against a Top1-DNA complex. Camptothecin demonstrates significant efficacy against gastric cancer, esophageal cancer, lung cancer, bladder cancer and the like, and is a broad-spectrum antitumor active drug. Camptothecin derivatives, irinotecan and topotecan, have been approved in many countries for the treatment of various cancers. Another camptothecin derivative, belotecan, has been approved in Korea for the treatment of SCLC and ovarian cancer. However, the main drawbacks of camptothecin-based antitumor drugs are their toxicity, poor solubility, and the development of drug resistance in tumor cells.

### SUMMARY

The present disclosure provides a series of 7-hydroxylamine-substituted alkyl camptothecin derivatives which contain a hydroxylamine structure. Such derivatives bearing an oxygen atom substituent on the nitrogen atom exhibit weak basicity, which leads to reduction in the hepatotoxicity of the compound in vivo and improvement in metabolic stability and cell membrane permeability, and is conducive to improving antitumor activity.

In one aspect, the present disclosure provides a compound of Formula (I), and a stereoisomer or a pharmaceutically acceptable salt thereof: 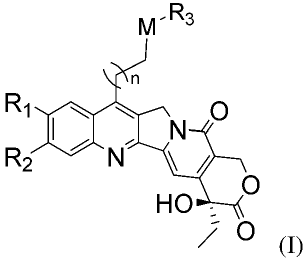
in the formula,
R₁ and R₂ are each independently selected from hydrogen, fluorine, and C₁₋₃ alkyl; or R₁ and R₂ together with the carbon atoms to which they are attached form an oxygen-containing heterocyclyl;
M is selected from -NH-O- and -O-NH-;
n is selected from 0, 1, 2, and 3; and
R₃ is selected from hydrogen, C₁₋₃ alkyl, or C₃₋₆ cycloalkyl.

In one embodiment, R₁ and R₂ are each independently selected from hydrogen, fluorine, and methyl.

In one embodiment, R₁ and R₂ together with the carbon atoms to which they are attached form heterocyclyl containing 1 or 2 oxygen atoms as ring members.

In one embodiment, R₁ and R₂ together with the carbon atoms to which they are attached form

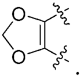

In one embodiment, R₁ is hydrogen, and R₂ is hydrogen;
R₁ is fluorine, and R₂ is fluorine;
R₁ is methyl, and R₂ is fluorine; or
R₁ and R₂ together with the carbon atoms to which they are attached form

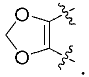

In one embodiment, R₃ is selected from C₁₋₃ alkyl;

In one embodiment, R₃ is methyl.

In one embodiment, the compound of Formula (I) has a structure as shown in Formula (IA) below: 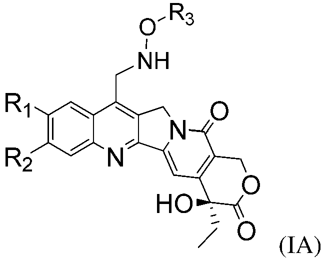
in the formula, R₁, R₂ and R₃ are each the same as defined for the compound of Formula (I);
preferably, R₁ is hydrogen, and R₂ is hydrogen;
R₁ is fluorine, and R₂ is fluorine;
R₁ is methyl, and R₂ is fluorine; or
R₁ and R₂ together with the carbon atoms to which they are attached form 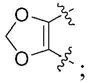 and
R₃ is methyl.

In one embodiment, the compound of Formula (I) has a structure as shown in Formula (IB) below:

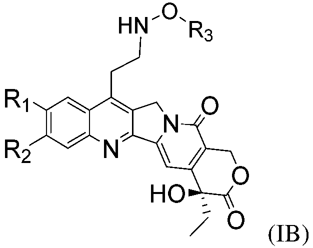

In the formula, R₁, R₂ and R₃ are each the same as defined for the compound of Formula (I);
preferably, R₁ is hydrogen, and R₂ is hydrogen;
R₁ is fluorine, and R₂ is fluorine;
R₁ is methyl, and R₂ is fluorine, or
R₁ and R₂ together with the carbon atoms to which they are attached form 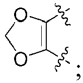 and
R₃ is methyl.

In one embodiment, the compound of Formula (I) has a structure as shown in Formula (IC) below:

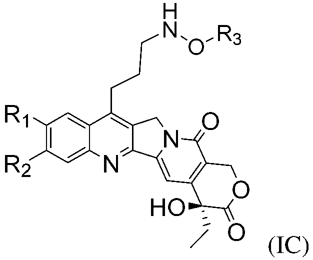

In the formula, R₁, R₂ and R₃ are each the same as defined for the compound of Formula (I);
preferably, R₁ is hydrogen, and R₂ is hydrogen;
R₁ is fluorine, and R₂ is fluorine;
R₁ is methyl, and R₂ is fluorine; or
R₁ and R₂ together with the carbon atoms to which they are attached form 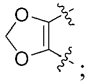 and
R₃ is methyl.

The present disclosure provides the following compounds, stereoisomers thereof, or pharmaceutically acceptable salts thereof:

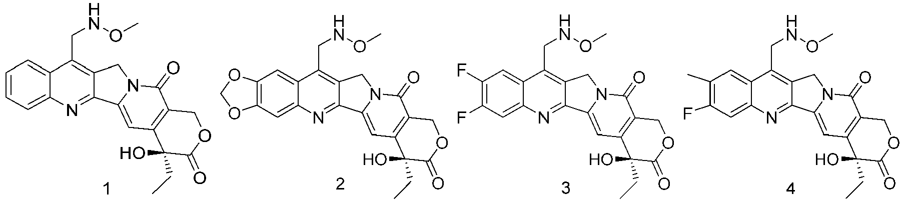

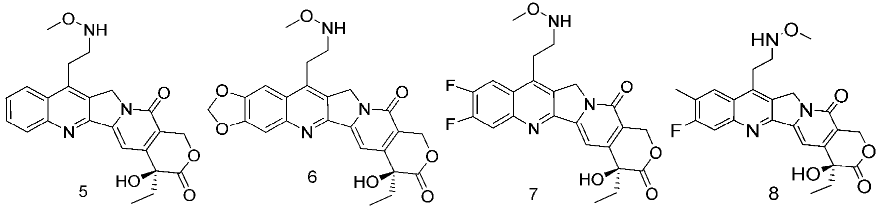

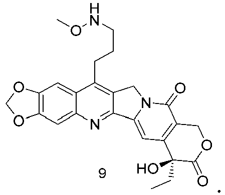

In another aspect, the present disclosure provides a preparation method of the above compound, wherein the preparation method is selected from the following synthetic routes:

### Synthetic route 1:

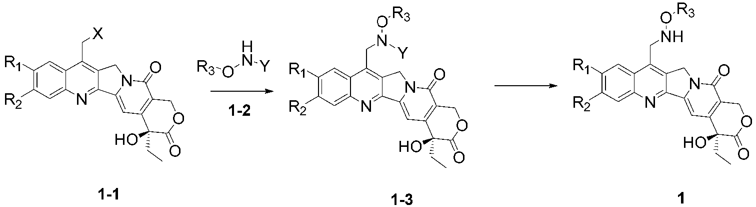

A compound of Formula (1-1) and a compound of Formula (1-2) are subjected to a substitution reaction to obtain a compound of Formula (1-3); and

Group Y is removed from the compound of Formula (1-3) to obtain the compound of Formula (1).

### Synthetic route 2:

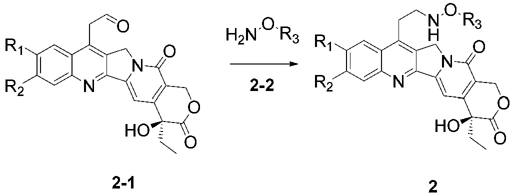

A compound of Formula (2-1) and a compound of Formula (2-2) are subjected to a reductive amination reaction to obtain a compound of Formula (2);

In the formula, R₁, R₂, and R₃ are each the same as defined for the compound of Formula (I);
X is selected from halogen, preferably bromine; and
Y is selected from amino protecting groups, preferably p-methoxybenzyl

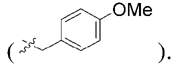

In one embodiment, R₃ is C₁₋₃ alkyl, and the preparation of the compound comprises one or more synthetic routes selected from the following:
subjecting corresponding 7-haloalkyl camptothecin and corresponding O-alkylhydroxylamine to a substitution reaction;
subjecting corresponding camptothecin-7-alkylaldehyde and corresponding O-alkylhydroxylamine to a reductive amination reaction;
subjecting corresponding camptothecin-7-alkylalcohol and corresponding N-Boc-N-alkylhydroxylamine to a Mitsunobu reaction; and
subjecting corresponding 7-haloalkyl camptothecin and corresponding N-Boc-N-alkylhydroxylamine to a substitution reaction.

The corresponding 7-haloalkyl camptothecin has a structure as shown in Formula (II) below:

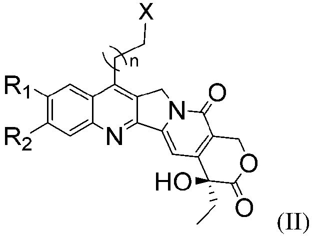

In the formula, the definitions of R₁, R₂ and n are the same as before; and
X is selected from halogen.

The corresponding camptothecin-7-alkylaldehyde has a structure as shown in Formula (III) below:

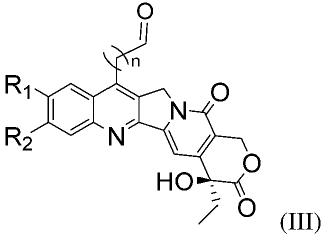

In the formula, the definitions of R₁, R₂ and n are the same as before.

The corresponding camptothecin-7-alkylalcohol has a structure as shown in Formula (IV) below:

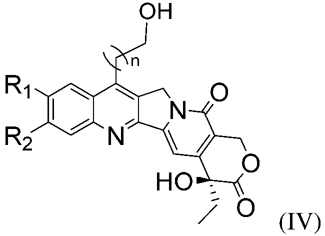

In the formula, the definitions of R₁, R₂ and n are the same as before.

In another aspect, the present disclosure provides a pharmaceutical composition, comprising the compound, the stereoisomer or the pharmaceutically acceptable salt thereof described above; and a pharmaceutically acceptable carrier.

In another aspect, the present disclosure provides use of the compound, the stereoisomer or the pharmaceutically acceptable salt thereof described above, or the pharmaceutical composition described above in the manufacture of an antitumor drug.

In one embodiment, the tumor is selected from solid tumor, more preferably selected from one or more of breast cancer, esophageal cancer, gastric cancer, and lung cancer.

In one embodiment, the compound can inhibit tumor growth.

In another aspect, the present disclosure provides a method for treating cancer, comprising a step of administering the compound, the stereoisomer or the pharmaceutically acceptable salt thereof described above, or the pharmaceutical composition described above to a patient in need thereof.

In one embodiment, the cancer is selected from one or more of breast cancer, esophageal cancer, gastric cancer, and lung cancer.

### DETAILED DESCRIPTION

### I. Definitions

In the present disclosure, unless otherwise specified, scientific and technical terms used herein have the meanings commonly understood by those skilled in the art. In addition, the related terms and laboratory operation steps used herein are terms and conventional steps widely used in the corresponding fields. Meanwhile, in order to better understand the present disclosure, the definitions and explanations of related terms are provided below.

As used herein and unless otherwise specified, the term "about" or "approximately" refers to a value within ±10% of the given value or range. When an integer is required, the term refers to rounding up or down to the nearest integer within ±10% of the given value or range.

The description herein involves "some examples", "some implementations," or "some embodiments", which describe a subset of all possible examples; however, it is to be understood that "some examples" may be the same subset or different subsets of all possible examples, and may be combined with each other without conflict.

As used herein and unless otherwise specified, the terms "comprising", "including", "having", "containing," as well as grammatical equivalent forms thereof are generally understood as open-ended and non-limiting, for example, not excluding other unlisted elements or steps.

The compounds described in the present application may be asymmetric, for example, having one or more stereoisomers. Unless otherwise specified, all stereoisomers are included, such as enantiomers and diastereomers. The stereoisomers include geometric isomers (e.g., cis and trans configurations) and optical isomers (e.g., enantiomers), as therapeutic agents consisting of individual isomers, racemates, racemic mixtures, and pharmaceutically acceptable salts thereof. Compounds containing asymmetric carbon atoms in the present application may be isolated in optically active pure form or racemic form. The optically active pure form may be resolved from a racemic mixture, or be synthesized by using chiral raw materials or chiral reagents. Racemates, diastereomers, and enantiomers are all encompassed within the scope of the present application.

The small molecule drugs used in the present application also include tautomeric forms. Tautomeric forms result from the exchange of a single bond with an adjacent double bond accompanied by the migration of a proton.

The numerical ranges herein refer to each individual integer within the given range. For example, "C₁₋₃" means that the group may have 1 carbon atom, 2 carbon atoms, or 3 carbon atoms; and "C₃₋₆" means that the group may have 3 carbon atoms, 4 carbon atoms, 5 carbon atoms, or 6 carbon atoms.

When any variable (e.g., Rₙ) appears more than once in the composition or structure of a compound, its definition in each instance is independent. Thus, for example, if a group is substituted with 1-5 R, the group may be optionally substituted with up to 5 R, and the R in each instance has independent options. In addition, combinations of substituents and/or variations thereof are permitted only if such combinations would result in stable compounds.

As used in the present application, the term "halogen" refers to fluorine, chlorine, bromine, and iodine.

As used in the present application, the term "alkyl" refers to a saturated aliphatic hydrocarbon group, which is a straight-chain or branched-chain group containing 1 to 20 carbon atoms, preferably an alkyl group containing 1 to 3 carbon atoms. Non-limiting examples include methyl, ethyl, n-propyl, and isopropyl.

As used in the present application, the term "heterocyclyl" or "heterocycloalkyl" refers to a saturated or partially unsaturated monocyclic or polycyclic hydrocarbon substituent containing 3 to 20 ring atoms, wherein one or more ring atoms are heteroatoms selected from nitrogen, oxygen, or S(O)ₘ (wherein m is an integer from 0 to 2), but excluding ring moieties containing -O-O-, -O-S-, or -S-S-, and the remaining ring atoms are carbon.

As used in the present application, the term "substituted" means that one or more hydrogen atoms in a group, preferably up to 5, more preferably 1 to 3 hydrogen atoms, are independently replaced by a corresponding number of substituents. It is understood that substituents are only located at their chemically feasible positions, and those skilled in the art can determine (by experiment or theory) possible or impossible substitutions without undue effort. For example, an amino or hydroxyl group having a free hydrogen may be unstable when bonded to a carbon atom having an unsaturated (e.g., olefinic) bond.

As used in the present application, the term "pharmaceutically acceptable salt" refers to a salt formed from a corresponding amine compound and an inorganic acid or organic acid, or a salt formed from a corresponding carboxylic acid compound and an alkali metal or alkaline earth metal, or a salt formed with an organic amine. The inorganic acids include, but are not limited to, hydrochloric acid, hydrobromic acid, hydroiodic acid, sulfuric acid, phosphoric acid, and the like; the organic acids include, but are not limited to, acetic acid, propionic acid, butyric acid, benzoic acid, methanesulfonic acid, benzenesulfonic acid, p-toluenesulfonic acid, oxalic acid, butanedioic acid, lactic acid, citric acid, succinic acid, gluconic acid, maleic acid, fumaric acid, tartaric acid, and the like; the alkali metal or alkaline earth metal salts include, but are not limited to, sodium, potassium, calcium, and magnesium salts; the organic amine salts include, but are not limited to, salts formed with ammonia, methylamine, ethylamine, propylamine, isopropylamine, dimethylamine, diethylamine, trimethylamine, triethylamine, tert-butylamine, ethylenediamine, ethanolamine, diethanolamine, triethanolamine, morpholine, piperidine, piperazine, amino acids, and the like.

As used in the present application, the term "pharmaceutical composition" means a composition comprising the compound described in the present application or a pharmaceutically acceptable salt thereof, and at least one pharmaceutically acceptable ingredient selected from the following and depending on the nature of the administration mode and dosage form, including but not limited to: carriers, diluents, adjuvants, excipients, preservatives, fillers, disintegrants, wetting agents, emulsifiers, suspending agents, sweeteners, flavoring agents, fragrances, antimicrobial agents, antifungal agents, lubricants, dispersants, temperature-sensitive materials, temperature regulators, adhesives, stabilizers, suspending agents, and the like.

The drug or pharmaceutical composition of the present application may be administered orally, topically, parenterally, or mucosally (e.g., buccally, by inhalation, or rectally) in dosage unit formulations comprising conventional non-toxic pharmaceutically acceptable carriers. The oral route is generally preferred. The active agent may be administered orally in the form of capsules, tablets, or the like (see Remington: The Science and Practice of Pharmacy, 20th Edition).

For oral administration in the form of tablets or capsules, the active pharmaceutical ingredient may be combined with non-toxic, pharmaceutically acceptable adjuvants such as binders (e.g., pregelatinized corn starch, polyvinylpyrrolidone, or hydroxypropyl methylcellulose); fillers (e.g., lactose, sucrose, glucose, mannitol, sorbitol and other reducing and non-reducing sugars, microcrystalline cellulose, calcium sulfate, or calcium hydrogen phosphate); lubricants (e.g., magnesium stearate, talcum power or silica, stearic acid, sodium stearyl fumarate, glycerol docosanoate, calcium stearate, and the like); disintegrants (e.g., potato starch or sodium starch glycolate); or wetting agents (e.g., sodium lauryl sulfate), coloring agents and flavoring agents, gelatin, sweeteners, natural and synthetic gums (such as acacia, tragacanth, or alginates), buffer salts, carboxymethylcellulose, polyethylene glycol, waxes, and the like. For oral administration in liquid form, the pharmaceutical ingredient may be combined with non-toxic, pharmaceutically acceptable inert carriers (e.g., ethanol, glycerol, water), anti-settling agents (e.g., sorbitol syrup, cellulose derivatives, or hydrogenated edible fats), emulsifiers (e.g., lecithin or acacia), non-water-based carriers (e.g., almond oil, oily esters, ethanol, or fractionated vegetable oils), preservatives (e.g., methyl p-hydroxybenzoate, propyl p-hydroxybenzoate, or sorbic acid), and the like. Stabilizers such as antioxidants (BHA, BHT, propyl gallate, sodium ascorbate, citric acid) may also be added to stabilize the dosage form.

Tablets comprising the active compound may be coated by methods well known in the art. The compositions of the present application comprising a compound of Formula (I) as the active compound may also be incorporated into beads, microspheres, or microcapsules, for example, constructed from polyglycolic acid/lactic acid (PGLA). Liquid formulations for oral administration may take the form of, for example, solutions, syrups, emulsions, or suspensions, or they may be presented as dry products for reconstitution with water or other suitable adjuvants before use. Formulations for oral administration may be suitably formulated to realize controlled or delayed release of the active compound.

The term "patient" refers to an animal, preferably a mammal, more preferably a human.

The term "effective amount" or "therapeutically effective amount" refers to a sufficient amount of a drug or agent that is non-toxic but achieves the desired effect. In the embodiments of the present disclosure, when treating a patient according to the present disclosure, the amount of a given drug depends on many factors, such as the specific administration regimen, the type and severity of the disease or disorders, and the uniqueness of the subject or host to be treated (e.g., body weight); however, the administration dose can be routinely determined by methods known in the art according to the specific surrounding circumstances, including, for example, the specific drug already used, the route of administration, the disorder being treated, and the subject or host being treated. Generally, for adult therapeutic use, the administration dose is typically in the range of 0.02-5000 mg/day, for example, about 1-1500 mg/day. The required dose may conveniently be presented as a single dose, or as divided doses administered simultaneously (or within a short period of time) or at appropriate intervals, for example, two, three, four, or more divided doses per day. Those skilled in the art will appreciate that although the above dose ranges are provided, the specific effective amount may be appropriately adjusted according to the patient's condition in combination with the physician's diagnosis.

As used in the present application, the term "treating" includes inhibiting, alleviating, preventing, or eliminating one or more symptoms or side effects associated with the disease, disorder, or imbalance being treated.

As used in the present application, the term "inhibiting" is used relative to a control. Those skilled in the art will easily determine appropriate controls for each experiment. For example, a reduced response in a subject or cell treated with the compound is compared to the response in a subject or cell not treated with the compound.

### II. Examples

In order to make the objects, technical solutions, and advantages of the present disclosure more clear, the present disclosure will be described in further detail below. The embodiments described should not be regarded as limiting the present disclosure. All other embodiments obtained by those of ordinary skill in the art without making inventive efforts fall within the scope of protection of the present disclosure.

Before providing further detailed description of the embodiments of the present disclosure, the nouns and terms involved in the embodiments of the present disclosure are explained as follows, and the nouns and terms involved in the embodiments of the present disclosure are applicable to the following interpretations.

The raw materials and equipment used in the specific embodiments of the present disclosure are all known products, which are obtained by purchasing commercially available products.

### Example 1: 7-(N-methoxy)aminomethylcamptothecin (1)

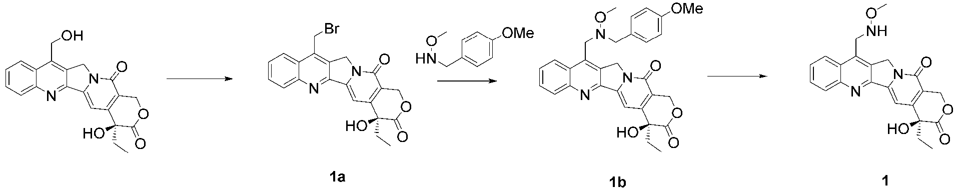

7-hydroxymethylcamptothecin (5 g, 12.4 mmol), a 50% aqueous hydrobromic acid solution (100 mL) and concentrated sulfuric acid (1 mL) were added to a reaction flask. The mixture was stirred and heated to 125°C, allowed to react for 2 h, then cooled to room temperature and poured into ice water. The precipitated solid was collected by filtration and dried to obtain 7-bromomethylcamptothecin **1a** (2.5 g, yield 26%); LCMS: [M+H]⁺ 441.05 (theoretical value 440.04).

7-(bromomethyl)camptothecin **1a** (1.20 g, 2.72 mmol), dimethyl formamide (DMF) (15 mL), and N-methoxy-(4-methoxybenzyl)amine (909.62 mg, 5.44 mmol) were added to a reaction flask. The reaction solution was stirred and heated to 55°C, allowed to react for 16 h, then poured into ice water. The precipitated solid was collected by filtration and purified by reverse-phase column chromatography to obtain 7-[N-methoxy-N-(4-methoxybenzyl)aminomethyl]camptothecin **1b** (700 mg, yield 44.3%); LCMS: [M+H]⁺ 528.32 (theoretical value 527.21).

7-[N-methoxy-N-(4-methoxybenzyl)aminomethyl]camptothecin (700 mg, 1.33 mmol), dichloromethane (DCM) (10 mL) and methanesulfonic acid (10 mL) were added to a reaction flask. The mixture was heated at 40°C and allowed to react for 96 h under stirring, and then concentrated. A saturated aqueous sodium bicarbonate solution was added to adjust the pH to 7, and the precipitated solid product was collected by filtration to obtain 7-(N-methoxy)aminomethylcamptothecin **1** (500 mg, yield 84.1%); LCMS: [M+H]⁺ 408.25 (theoretical value 407.15); ¹H NMR (600 MHz, DMSO-d₆) δ 8.33 (d, J=8.6 Hz, 1H), 8.13 (d, J=8.5 Hz, 1H), 7.82 (dd, J=8.3, 6.5 Hz, 1H), 7.69 (ddd, J=8.3, 6.7, 1.5 Hz, 1H), 7.33 (s, 1H), 7.24 (t, J=5.8 Hz, 1H), 5.43 (d, J=2.1 Hz, 2H), 5.34 (s, 2H), 4.54 (d, J=5.6 Hz, 1H), 3.29 (s, 2H), 2.35 (s, 3H), 1.92-1.84 (m, 2H), 0.89 (t, J=7.2 Hz, 3H); ¹³C NMR (151 MHz, DMSO) δ 172.95, 157.27, 152.34, 150.53, 148.91, 146.05, 140.26, 130.44, 130.03, 129.96, 127.92, 127.61, 125.26, 119.54, 97.20, 72.87, 65.74, 61.50, 50.49, 49.19, 49.07, 30.79.

### Example 2: 7-(N-methoxy)aminomethyl-10,11-methylenedioxycamptothecin (2)

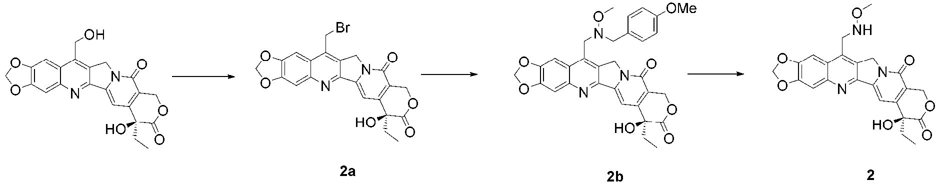

At 0°C, 7-hydroxymethyl-10,11-methylenedioxycamptothecin (2.5 g, 5.90 mmol), a 48% aqueous hydrobromic acid solution (477.76 mg, 5.90 mmol, 60 mL) and concentrated sulfuric acid (1 mL) were added to a reaction flask. The mixture was further stirred at 0°C to react for 30 min, and then poured into ice water. The precipitated solid was collected by filtration and dried to obtain a brown solid product 7-bromomethyl-10,11-methylenedioxycamptothecin **2a** (1.7 g, yield 46.2%); LCMS: [M+H]⁺ 485.20 (theoretical value 484.03).

7-(bromomethyl)-10,11-methylenedioxycamptothecin **2a** (1.7 g, 2.73 mmol) and N-methoxy-(4-methoxybenzyl)amine (1.07 g, 6.37 mmol) were added to a reaction flask. The reaction solution was subjected to reaction at 55°C under stirring for 8 h, and then poured into ice water. The mixture was extracted with ethyl acetate, washed with saturated brine, dried over anhydrous sodium sulfate, and purified by silica gel column chromatography to obtain 7-[(N-methoxy-N-(4-methoxyphenyl)amino)methyl]-10,11- methylenedioxycamptothecin **2b** (1.1 g, yield 55.5%); LCMS: [M+H]⁺ 572.28 (theoretical value 571.20); ¹H NMR (500 MHz, DMSO-d₆) δ 7.52 (s, 1H), 7.47 (s, 1H), 7.35 (d, J=8.6 Hz, 2H), 7.23 (s, 1H), 6.94 (d, J=8.6 Hz, 2H), 6.29 (s, 2H), 5.42 (s, 2H), 5.19 (s, 2H), 4.26 (s, 2H), 4.00 (s, 2H), 3.76 (s, 3H), 2.80 (s, 3H), 1.96-1.78 (m, 2H), 0.89 (t, J=7.3 Hz, 3H).

7-[(N-methoxy-N-(4-methoxyphenyl)amino)methyl]-10,11-methylenedioxycamptothecin **2b** (1 g, 1.75 mmol) and trifluoroacetic acid (50 mL) were added to a reaction flask. The reaction solution was allowed to react at 40°C overnight under stirring, concentrated, and purified by silica gel column chromatography to obtain 7-(N-methoxy)aminomethyl-10,11-methylenedioxycamptothecin **2** (260 mg, yield 32.3%); LCMS: [M+H]⁺ 452.34 (theoretical value 451.14); ¹H NMR (500 MHz, DMSO-d₆) δ 7.66 (s, 1H), 7.50 (d, J=3.0 Hz, 1H), 7.24 (s, 1H), 6.49 (s, 1H), 6.29 (d, J=2.4 Hz, 2H), 5.42 (d, J=1.7 Hz, 2H), 5.27 (s, 2H), 4.44 (d, J=3.7 Hz, 2H), 3.30 (s, 3H), 1.87 (dp, J=17.2, 7.1 Hz, 2H), 0.89 (t, J=7.3 Hz, 3H); ¹³C NMR (126 MHz, DMSO) δ 173.00, 157.28, 151.35, 150.59, 149.86, 149.23, 147.71, 146.49, 138.83, 128.94, 125.24, 118.60, 105.69, 103.10, 100.62, 96.39, 72.88, 65.71, 61.53, 50.42, 49.54, 30.70, 8.26.

### Example 3: 7-(N-Methoxy)aminomethyl-10,11-difluorocamptothecin (3)

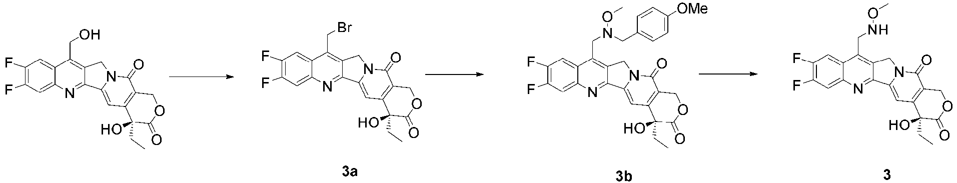

7-hydroxymethyl-10,11-difluorocamptothecin (6 g, 14.45 mmol), a 48% aqueous hydrobromic acid solution (150 mL) and concentrated sulfuric acid (1 mL) were added to a reaction flask. The reaction solution was stirred and heated to 125°C to react for 1.5 h, and then poured into water. The precipitated solid was collected by filtration and dried to obtain 7-bromomethyl-10,11-difluorocamptothecin **3a** (4.5 g, yield62%); LCMS: [M+H]⁺ 477.09, [M+3]⁺ 479.04 (theoretical value 476.02).

7-bromomethyl-10,11-difluorocamptothecin **3a** (4.5 g, 4.70 mmol), DMF (80 mL) and N-methoxy-(4-methoxybenzyl)amine (1.58 g, 9.41 mmol) were added to a reaction flask. The mixture was stirred and heated to 50°C to react for 4 h, and then poured into water. The mixture was extracted with DCM, washed with saturated brine, dried over anhydrous magnesium sulfate, and purified by silica gel column chromatography to obtain a yellow solid product 7-(N-methoxy-N-(4-methoxyphenyl)aminomethyl)-10,11-difluorocamptothecin **3b** (1.6 g, yield 58.4%); LCMS: [M+H]⁺ 564.17 (theoretical value 563.19).

7-(N-methoxy-N-(4-methoxyphenyl)aminomethyl)-10,11-difluorocamptothecin **3b** (120 mg, 212.56 µmol) was dissolved in methanesulfonic acid (702.74 mg, 10.63 mmol, 3 mL). The reaction solution was allowed to react at 40°C for 120 h, and a saturated aqueous sodium bicarbonate solution was added. The precipitated solid was collected by filtration and purified by silica gel column chromatography to obtain a yellow solid product 7-(N-methoxy)aminomethyl-10,11-difluorocamptothecin **3** (45 mg, yield 47.2%); LCMS: [M+H]⁺ 444.64 (theoretical value 443.13); ¹H NMR (500 MHz, DMSO-d₆) δ 8.36 (dd, J=12.4, 8.7 Hz, 1H), 8.16 (dd, J=11.5, 8.0 Hz, 1H), 7.31 (s, 1H), 6.53 (s, 1H). 5.43 (s, 2H), 5.35 (s, 2H), 4.53 (s, 2H), 3.30 (s, 3H), 1.87 (ddt, J=16.9, 14.1, 7.1 Hz, 2H), 1.39-1.26 (m, 2H), 1.28-1.19 (m, 5H), 0.87 (dt, J=21.6, 7.2 Hz, 3H), 0.84 (s, 1H); ¹³C NMR (126 MHz, DMSO-d6) δ 174.96, 172.87, 157.15, 153.14, 153.12, 152.92, 152.79, 150.91, 150.78, 150.45, 148.92, 148.79, 146.47, 146.38, 145.56, 140.71, 140.67, 130.46, 125.25, 125.18, 124.79, 124.71, 119.90, 116.19, 116.07, 112.10, 111.95, 97.39, 72.81, 65.71, 61.51, 50.48, 49.33, 34.95, 34.12, 31.75, 31.60, 30.77, 30.28, 29.88, 29.48, 29.36, 29.20, 29.16, 29.01, 24.95, 22.55, 14.40, 8.21.

### Example 4: 7-(N-(methoxy)amino)methyl-10-methyl-11-fluorocamptothecin (4)

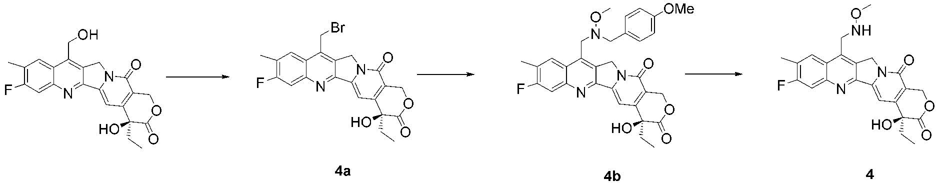

At 0°C, 7-hydroxymethyl-10-methyl-11-fluorocamptothecin (4 g, 9.72 mmol), a 48% aqueous hydrobromic acid solution (30 mL) and concentrated sulfuric acid (0.9 mL) were added to a reaction flask. The mixture was stirred at 125°C to react for 30 min, and then poured into ice water. The precipitated solid was collected by filtration and dried to obtain a brown solid product 7-bromomethyl-10-methyl-11-fluorocamptothecin **4a** (5.5 g, yield 84%); LCMS: [M+H]⁺ 473.26 (theoretical value 472.04).

7-(bromomethyl)-10-methyl-11-fluorocamptothecin **4a** (5.5 g, 11.60 mmol), DMF (50 mL) and N-methoxy-(4-methoxybenzyl)amine (3.90 g, 23.19 mmol) were added to a reaction flask. The reaction solution was stirred at 55°C to react for 6 h, and then poured into ice water. The mixture was extracted with ethyl acetate, washed with saturated brine, dried over anhydrous sodium sulfate, and purified by silica gel column chromatography to obtain 7-[(N-methoxy-N-(4-methoxyphenyl)amino)methyl]-10-methyl-11-fluorocamptothecin **4b** (1.91 g, yield 27.3%); LCMS: [M+H]⁺ 560.24 (theoretical value 559.21); ¹H NMR (500 MHz, DMSO-d₆) δ 8.00 (d, J=8.3 Hz, 1H), 7.85 (d, J=10.7 Hz, 1H), 7.42-7.36 (m, 2H), 7.31 (s, 1H), 7.01-6.93 (m, 2H), 5.44 (s, 2H), 5.29 (s, 2H), 4.34 (t, J=9.9 Hz, 2H), 4.03 (s, 2H), 3.77 (s, 3H), 2.85 (s, 3H), 2.44 (s, 3H), 1.87 (ddt, J=17.3, 14.0, 7.1 Hz, 2H), 0.89 (t, J=7.3 Hz, 3H).

7-[(N-methoxy-N-(4-methoxyphenyl)amino)methyl]-10-methyl-11-fluorocamptothecin **4b** (1.9 g, 3.39 mmol) was dissolved in methanesulfonic acid (20 mL). The reaction solution was stirred at 40°C to react for 168 h. A saturated aqueoussodium bicarbonate solution was added, and the precipitated solid was collected by filtration, and purified by silica gel column chromatography to obtain a yellow solid product 7-(N-methoxy)amino)methyl-10-methyl-11-fluorocamptothecin **4** (390 mg, yield 24.9%); LCMS: [M+H]⁺ 440.08 (theoretical value 439.15); ¹H NMR (500 MHz, DMSO-d₆) δ 8.22 (d, J=8.2 Hz, 1H), 7.80 (d, J=10.7 Hz, 1H), 7.29 (s, 1H), 7.23 (t, J=5.7 Hz, 1H), 6.52 (s, 1H), 5.42 (s, 2H), 5.27 (s, 2H), 4.51 (d, J=5.6 Hz, 2H), 3.30 (s, 3H), 2.47 (s, 3H), 1.87 (dh, J=21.4, 7.2 Hz, 2H), 0.90 (t, J=7.3 Hz, 3H); ¹³C NMR (126 MHz, DMSO) δ 172.91, 162.24, 157.18, 152.52, 150.46, 148.92, 145.83, 139.90, 129.45, 127.58, 127.19, 124.77, 119.55, 112.71, 97.18, 72.84, 65.72, 55.37, 50.43, 49.20, 30.77, 15.67, 8.24.

### Example 5: 7-(N-(methoxy)amino)ethylcamptothecin (5)

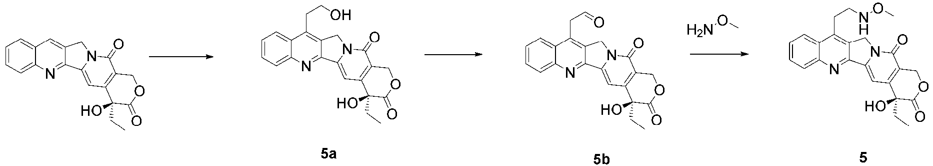

At 0°C, camptothecin (1 g, 2.86 mmol) and FeSO₄·7H₂O (795.78 mg, 2.86 mmol) were dissolved in H₂O (32.5 mL) in a 100 mL round-bottom flask. H₂SO₄ (17.5 mL) and 1,3-propanediol (10.59 g, 137.39 mmol, 10.06 mL) were added, and then a 35% aqueoushydrogen peroxide solution (97.36 mg, 2.86 mmol, 87.71 µL) was added dropwise. The mixture was allowed to react at 0°C for 3 h, and then poured into ice water. The solid product was collected by filtration and purified by normal-phase silica gel column chromatography to obtain a pale yellow powdery solid product 7-(2-hydroxyethyl)camptothecin **5a** (345 mg, yield 29%); LCMS: [M+1]⁺ 393.43 (calculated value 392.41); ¹H NMR (500 MHz, DMSO-d₆) δ 8.23 (d, J=8.4 Hz, 1H), 8.13 (d, J=8.4 Hz, 1H), 7.81 (t, J=7.6 Hz, 1H), 7.67 (t, J=7.6 Hz, 1H), 7.31 (s, 1H), 5.43 (s, 2H), 5.25 (s, 2H), 3.81 (t, J=6.3 Hz, 3H), 3.35 (t, J=6.4 Hz, 2H), 1.98-1.80 (m, 2H), 0.90 (t, J=7.3 Hz, 3H).

At 25°C, **5a** (345 mg, 0.833 mmol) was dissolved in dimethylsulfoxide (DMSO) (20 mL) and DCM (6 mL) in a 250 mL round-bottom flask. Under stirring and ice-bath cooling, Dess-Martin periodinane (706 mg, 1.666 mmol) was added. The ice bath was removed, and the mixture was stirred at room temperature to react for 3 h. Water (100 mL) was added, followed by extraction with dichloromethane (3×100 mL). The DCM organic phases were combined, dried over anhydrous magnesium sulfate and filtered. The filtrate was concentrated under reduced pressure to obtain a brown viscous solid 7-(N-(methoxy)amino)ethylcamptothecin **5b** (291 mg, 0.74 mmol, 85% yield) which was a brown viscous solid and was used in the next step without further purification; LCMS: [M+H]⁺ 391.23 (calculated value 390.40).

**5b** (200 mg, 0.512 mmol), EtOH (5 mL), (O-methyl)hydroxylamine hydrochloride (100.2 mg, 1.2 mmol) and sodium acetate (100.8 mg, 1.2 mmol) were added to a reaction flask. The mixture was stirred at room temperature for 6 h, acetic acid (15 mL) and sodium cyanoborohydride (94.5 mg, 1.5 mmol) were added, and the mixture was allowed to react at room temperature for 2 h. Water (100 mL) was added, and the mixture was extracted with dichloromethane (100 mL×4). The organic phases were combined, dried over anhydrous magnesium sulfate, and filtered. The filtrate was concentrated under reduced pressure and purified by reverse-phase C18 column chromatography (acetonitrile (ACN)/0.1% aqueous trifluoroacetic acid (TFA)). The collected solutions were lyophilized to obtain a pale yellow powdery solid product **5** 7-(N-methoxy)aminoethylcamptothecin (80 mg, yield 37%); LCMS: [M+1]⁺ 422.23 (calculated value 421.45); ¹H NMR (500 MHz, DMSO-d₆) δ 8.28 (d, J=8.5 Hz, 1H), 8.20 (d, J=8.4 Hz, 1H), 7.88 (t, J=7.6 Hz, 1H), 7.77 (t, J=7.7 Hz, 1H), 7.35 (s, 1H), 6.72-6.44 (m, 1H), 5.45 (s, 2H), 5.36 (s, 2H), 3.54 (s, 3H), 3.46-3.41 (m, 2H), 3.29 (t, J=7.3 Hz, 2H), 1.92-1.85 (m, 2H), 0.88 (d, J=7.4 Hz, 3H).

### Example 6: 7-(N-methoxy)aminoethyl-10,11-methylenedioxycamptothecin (6)

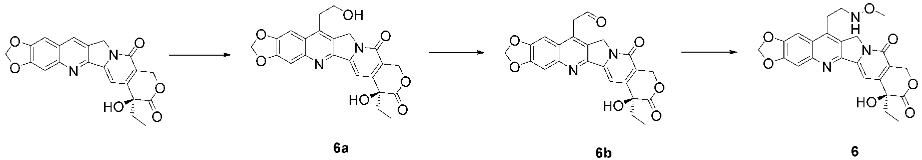

Under an ice bath, water (25 mL), 75% dilute sulfuric acid (25 mL) and 1,3-propanediol (25 mL) were sequentially added to a 250 mL three-necked eggplant-shaped flask. After stirring until homogeneous, 10,11-methylenedioxycamptothecin (10 g, 25.5 mmol) was added, followed by stirring until dissolved. Ferrous sulfate heptahydrate (14.17 g, 51 mmol) was then added and stirred until homogeneous. A 30% aqueous hydrogen peroxide solution (28.9 g, 0.25 mol) was added dropwise slowly while maintaining the temperature below 10°C and stirring to react for 10 min. The reaction solution was quenched by pouring into ice water, and the precipitated solid was collected by filtration. The filter cake was rinsed once with ethanol. The filter cake was then directly mixed with silica gel and methanol for sample loading, and was purified by silica gel column chromatography to obtain an off-white solid product7-(2-hydroxyethyl)-10,11-methylenedioxycamptothecin **6a** (2.2 g, yield 19.8%). LCMS: [M+1]⁺ 437.32 (calculated value 436.42); ¹H NMR (500 MHz, DMSO-d₆) δ 7.64 (s, 1H), 7.52 (s, 1H), 7.28 (s, 1H), 6.54 (s, 1H), 6.33 (s, 2H), 5.47 (s, 2H), 5.25 (s, 2H), 3.82 (d, J=6.4 Hz, 1H), 3.29 (t, J=5.9 Hz, 2H), 1.92 (p, J=7.8, 7.4 Hz, 2H), 1.75 (d, J=6.4 Hz, 1H), 0.94 (t, J=7.3 Hz, 3H).

7-(2-hydroxyethyl)-10,11-methylenedioxycamptothecin (400 mg, 0.9 mmol), DCM (20 mL) and DMSO (20 mL) were added sequentially to a 100 mL single-necked eggplant-shaped flask. After stirring until homogeneous, Dess-Martin periodinane (971.9 mg, 2.3 mmol) was added and the mixture was allowed to react under stirring for 10 min. Water (50 mL) was added. The mixture was extracted with DCM (3 × 100 mL). The layers were separated, and the organic phases were combined. The combined organic phase was washed with saturated aqueous sodium chloride, dried over anhydrous magnesium sulfate, filtered, and concentrated to obtain a brownish-yellow solid crude product **6b** 10,11-methylenedioxycamptothecin-7-acetaldehyde which was used directly in the next reaction without further purification; LCMS: [M+1]⁺ 435.59 (calculated value 434.40).

At room temperature, the aforementioned 10,11-methylenedioxycamptothecin-7-acetaldehyde **6b** (crude product) and ethanol (8 mL) were added to a reaction flask and stirred until homogeneous. Sodium acetate (755.4 mg, 0.9 mmol) and hydroxylamine hydrochloride (789 mg, 0.9 mmol) were then added, and the mixture was stirred for 1 h. Subsequently, acetic acid (24 mL) and sodium cyanoborohydride (1.16 g, 18 mmol) were added sequentially to the reaction solution, and the mixture was stirred to react for 1 h. Water (100 mL) was then added to the reaction solution. The mixture was extracted with DCM twice, washed with water, washed with a saturated aqueous sodium chloride solution, dried over anhydrous sodium sulfate, and concentrated to obtain a product 7-(N-methoxy)aminoethyl-10,11-methylenedioxycamptothecin **6** (320 mg; total yield for the two steps: 76%). LCMS: [M+1]⁺ 466.18 (calculated value 465.46); ¹H NMR (500 MHz, DMSO-d₆) δ 7.54 (s, 1H), 7.46 (s, 1H), 7.22 (s, 1H), 6.49 (s, 1H), 6.28 (d, J=2.7 Hz, 2H), 5.42 (d, J=2.6 Hz, 2H), 5.15 (s, 2H), 3.45 (s, 3H), 3.22 (t, J=7.1 Hz, 2H), 3.12 (t, J=7.1 Hz, 2H), 1.87 (dh, J=22.0, 7.4 Hz, 2H), 0.89 (t, J=7.3 Hz, 3H); ¹³C NMR (126 MHz, DMSO-d₆) δ 173.00, 157.27, 151.26, 150.57, 149.75, 149.35, 147.50, 146.82, 128.54, 124.84, 118.45, 105.94, 103.09, 99.76, 96.34, 72.87, 65.72, 61.10, 50.24, 50.17, 30.74, 28.00, 8.26.

### Example 7: 7-(N-methoxy)aminoethyl-10,11-difluorocamptothecin (7)

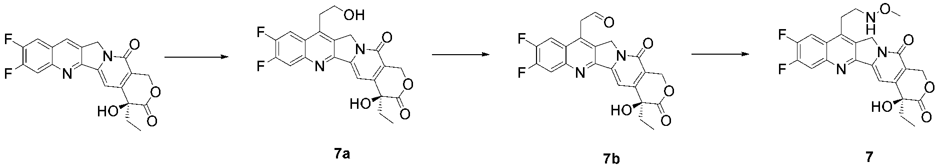

At 0°C, 10,11-difluorocamptothecin (3 g, 7.37 mmol), H₂O (75 mL) and 1,3-propanediol (62.07 g, 626.45 mmol, 58.95 mL) were added to a 500 mL three-necked reaction flask. Subsequently, 75% H₂SO₄ (36.14 g, 368.50 mmol, 75 mL) was added. At 0°C, ferrous sulfate heptahydrate (2.34 g, 8.40 mmol) was added, followed by dropwise addition of a 30% H₂O₂ aqueous solution (215.59 mg, 6.34 mmol, 194.23 µL). The mixture was further stirred at 0°C for 4 h. The reaction solution was then poured into 2 L of ice water and allowed to stand for 16 h. The precipitated solid was collected by filtration and dried to obtain 7-(2-hydroxyethyl)-10,11-difluorocamptothecin **7a** (1.8 g, yield 54.1%); LCMS: [M+1]⁺ 429.31 (theoretical value 428.39).

In a 100 mL three-necked reaction flask, 7-(2-hydroxyethyl)-10,11-difluorocamptothecin (1 g, 2.22 mmol), DMSO (20 mL) and DCM (14 mL) were added. Dess-Martin periodinane (1.88 g, 4.44 mmol) was then added, and the mixture was stirred at 25°C for 4 h. Water (50 mL) was added, and the mixture was extracted with DCM (3 × 100 mL), washed with a saturated sodium chloride aqueous solution, dried over anhydrous sodium chloride, filtered, and concentrated to obtain a brownish-yellow solid crude product **7b** 10,11-difluorocamptothecin-7-acetaldehyde which was used directly in the next reaction step without further purification; LCMS: [M+1]⁺ 427.16 (theoretical value 426.38).

10,11-difluorocamptothecin-7-acetaldehyde (50 mg, 111.27 µmol), DMSO (6 mL) and DCM (4 mL) were added to a 100 mL reaction flask. Subsequently, (O-methyl)hydroxylamine hydrochloride (23.38 mg, 333.81 µmol) was added, and the mixture was stirred at 25°C for 1 h. NaBH₃CN (13.98 mg, 222.54 µmol) and glacial acetic acid (33.41 mg, 556.35 µmol) were then added, and the mixture was stirred at 25°C for 16 h. The reaction solution was concentrated and purified using a C18 Spherical 20-35 µm 100A 120 g reverse-phase column. Acetonitrile and a 0.1% TFA acid aqueous were used as mobile phase B2 and mobile phase A2, respectively. HPLC preparation method (monitoring at wavelengths 254 nm and 214 nm): 2 mL of sample DMF solution was injected onto the reverse-phase column; the DMF solvent was flushed out within 5 min (a mobile phase ratio A2:B2=95:5); the proportion of acetonitrile was then increased to 10% within 90 min (changing the ratio from A2:B2=95:5 to A2:B2=90:10); finally, the acetonitrile proportion was maintained at 10% for an additional 30 min to elute the product, so as to obtain 7-(N-methoxy)aminoethyl-10,11-difluorocamptothecin **7** (13.5 mg, yield 26.5%). LCMS: [M+1]⁺ 458.20 (theoretical value 457.43); ¹H NMR (600 MHz, DMSO-d₆) δ 8.35-8.25 (m, 1H), 8.21-8.13 (m, 1H), 7.31 (s, 1H), 6.53 (s, 1H). 5.44 (s, 2H), 5.30 (s, 2H), 3.42 (s, 2H), 3.16 (t, J=6.5 Hz, 2H), 1.87 (dq, J=14.1, 6.8 Hz, 2H), 1.23 (s, 3H), 0.88 (t, J=7.2 Hz, 3H).

### Example 8: 7-(N-methoxy)aminoethyl-10-methyl-11-fluorocamptothecin (8)

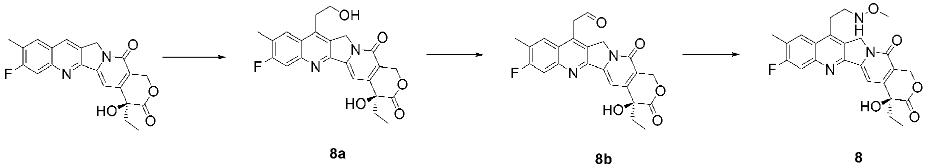

Water (2.5 mL), 75% sulfuric acid (2.5 mL), 1,3-propanediol (2.5 mL), 10-methyl-11-fluorocamptothecin (500 mg, 1.3 mmol) and ferrous sulfate heptahydrate (722.8 mg, 2.6 mmol) were added to a 100 mL three-necked flask. The mixture was dissolved by stirring and then cooled to 0°C, and a 30% H₂O₂ aqueous solution (1.7 g, 26 mmol) was added dropwise. The reaction was maintained at about 15°C for 1 h. The reaction solution was then poured into water, filtered and dried to obtain 7-(2-hydroxyethyl)-10-methyl-11-fluorocamptothecin **8a** (311 mg, yield 39.8%); LCMS: [M+1]⁺ 425.35 (calculated value 424.41).

7-(2-hydroxyethyl)-10-methyl-11-fluorocamptothecin (300 mg, 0.7 mmol) was added to a 100 mL single-necked flask, dissolved in DCM/DMSO (1:1, 30 mL), and then Dess-Martin periodinane (742 mg, 1.75 mmol) was added. The mixture was allowed to react at room temperature for 10 min, and extracted with DCM (50 mL× 3); the organic phases were combined, washed with saturated brine, and dried over anhydrous sodium sulfate and then evaporated to dryness to obtain 10-methyl-11-fluorocamptothecin-7-acetaldehyde **8b** (280 mg, crude yield 95%) which was used directly in the next reaction step without further purification; LCMS: [M+1]⁺ 423.35 (calculated value 422.41).

The aforementioned 10-methyl-11-fluorocamptothecin-7-acetaldehyde **8b** (150 mg, 0.35 mmol) dissolved in EtOH (4 mL) was added to a 100 mL single-necked flask. Under stirring, (O-methyl)hydroxylamine hydrochloride (292 mg, 3.5 mmol) and sodium acetate (287 mg, 3.5 mmol) were added. The mixture was allowed to react at room temperature for 30 min, sodium cyanoborohydride (287 mg, 53 mmol) was added to the reaction solution, and glacial acetic acid (12 mL) was added. The mixture was stirred at room temperature for 1 h, and then water (12 mL) was added. The mixture was extracted with DCM, washed with saturated brine, dried over anhydrous sodium sulfate, concentrated, purified using a C18 reverse-phase column, and lyophilized to obtain a yellow solid product 7-(N-methoxy)aminoethyl-10-methyl-11-fluorocamptothecin **8** (10 mg, yield 6.8%); LCMS: [M+1]⁺ 454.45 (calculated value 453.47); ¹H NMR (500 MHz, DMSO-d₆) δ 8.18 (d, J=8.3 Hz, 1H), 7.85 (d, J=10.8 Hz, 1H), 7.30 (s, 1H), 6.52 (s, 1H), 5.43 (s, 2H), 5.27 (s, 2H), 3.28-3.40 (s, 6H), 3.17 (t, J=6.9 Hz, 2H), 2.51 (s, 3H), 2.03-1.78 (m, 2H), 0.88 (t, J=7.3 Hz, 3H).

### Example 9: 7-(N-methoxy)aminopropyl-10,11-methylenedioxycamptothecin (9)

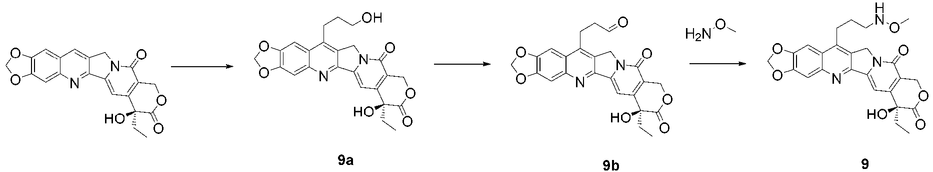

Under ice-bath cooling, purified water (12.5 mL), 75% dilute sulfuric acid (12.5 mL) and 1,4-butanediol (12.5 mL) were sequentially added to a 100 mL three-necked flask. After stirring until homogeneous, 10,11-methylenedioxycamptothecin (5 g, 12.7 mmol) was added, and the mixture was stirred until dissolved (internal temperature maintained at about 10°C). Ferrous sulfate heptahydrate (7.4 g, 26.6 mmol) was then added; after stirring until homogeneous, 30% aqueous H₂O₂ (14.5 g, 127.9 mmol) was slowly added dropwise. The mixture was allowed to react under stirring for 10 min while maintaining the temperature below 10°C. The reaction solution was then quenched by pouring it into ice water and subjected to suction filtration. The filter cake was rinsed once with ethanol. The rinsed filter cake was directly mixed with silica gel and methanol for sample loading. The mixture obtained after sample loading was purified by silica gel column chromatography to obtain a pale yellow solid product **9a** 7-(3-hydroxypropyl)-10,11-methylenedioxycamptothecin (1.56 g, yield 27%); LCMS: [M+1]⁺ 451.6 (calculated value 450.45).

7-(3-hydroxypropyl)-10,11-methylenedioxycamptothecin **9a** (400 mg, 0.89 mmol), 20 mL of DCM, and 20 mL of DMSO were sequentially added to a 100 mL single-necked eggplant-shaped flask. After stirring until homogeneous, Dess-Martin periodinane (753 mg, 1.78 mmol) was added, and the mixture was stirred at room temperature to react for 10 min. Water (10 mL) was then added to the reaction solution. The mixture was extracted with DCM (3 × 50 mL). The layers were separated, and the organic phases were combined. The combined organic phase was washed with a saturated aqueous sodium chloride solution, dried over anhydrous sodium sulfate, and concentrated to obtain a brownish-yellow crude solid product **9b** which was used directly in the next reaction step without further purification; LCMS: [M+1]⁺ 449.27 (calculated value 448.43).

At room temperature, ethanol (10 mL) was added to the aforementioned compound **9b,** followed by the addition of sodium acetate (1.09 g, 13.3 mmol) and (O-methyl)hydroxylamine hydrochloride (924.2 mg, 13.3 mmol). The mixture was stirred for 1 h, 24 mL of acetic acid and sodium cyanoborohydride (1.11 g, 17.7 mmol) were added, and the reaction was carried out under stirring for 1 h. Water (100 mL) was then added to the reaction solution, and the mixture was extracted with DCM (2 × 50 mL). The organic phase was washed once with water and once with a saturated aqueous sodium chloride solution, dried over anhydrous sodium sulfate, concentrated, and purified by preparative reverse-phase chromatography to obtain 7-(N-methoxy)aminopropyl-10,11-methylenedioxycamptothecin **9** (120 mg, yield 13%). LCMS: [M+1]⁺ 480.31 (calculated value 479.49); ¹H NMR (500 MHz, DMSO-d₆) δ 7.61 (s, 1H), 7.47 (s, 1H), 7.22 (s, 1H), 6.28 (d, J=2.6 Hz, 2H), 5.41 (d, J=2.7 Hz, 2H), 5.14 (s, 2H), 3.72 (s, 3H), 3.29 (t, J=7.2 Hz, 2H), 3.17-3.09 (m, 2H), 1.93-1.83 (m, 4H), 0.88 (t, J=7.3 Hz, 3H).

### Test example 1: Inhibition of tumor cell growth activity

Human esophageal cancer cells (OE33), human breast cancer cells (MDA-MB-231), human gastric cancer cells (NCI-N87), human lung cancer cells (NCI-H1975), human esophageal cancer cells (TE12) and the like were cultured in RPMI 1640 medium (Cellmax) containing 10% fetal bovine serum (Cellmax). Tumor cells in the exponential growth phase were diluted with culture medium to a density of 1 × 10⁵ cells/mL; 100 µL of cell suspension was added to a 96-well cell culture plate, and the plate was returned to a 37°C, 5% CO₂ incubator for overnight incubation. On the following day, the compound was diluted with a culture medium to final concentrations of 10,000 nM, 2,000 nM, 400 nM, 80 nM, 16 nM, 3.2 nM, 0.64 nM, and 0.13 nM. 2 µL of diluted compound was added to the 96-well cell culture plate, with three replicate wells assigned to each concentration. For negative control (with no compound) and blank control groups, 2 µL of diluted solution was added to each well. Dxd was tested at the same concentrations to serve as positive control for screening. After the sample addition was completed, the plate was returned to the 37°C, 5% CO₂ incubator for 72 h of continued incubation. After completion of the incubation, the cell culture plate was taken out, and the culture medium in the culture plate was aspirated and removed using a pipette. 100 µL of culture medium containing 10% CCK-8 was then added to each well, followed by incubation at 37°C for 3 h. After completion of the incubation, the plate was taken out, shielded from light, and placed in a microplate reader to measure absorbance by using 630 nm as the reference wavelength and 450 nm as the measurement wavelength. Based on the absorbance values, IC₅₀ values were calculated using four-parameter regression analysis in GraphPad (Table 1).

The structural formula of Dxd is

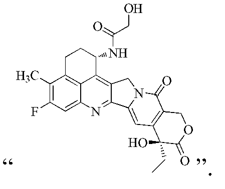

Regarding IC₅₀ values, wherein "++++" indicates 10 nM>IC₅₀; "+++" indicates 100 nM>IC₅₀ ≥ 10 nM; "++" indicates 500 nM>IC₅₀≥100 nM ; and "+" indicates 1000 nM>IC₅₀≥500 µM.

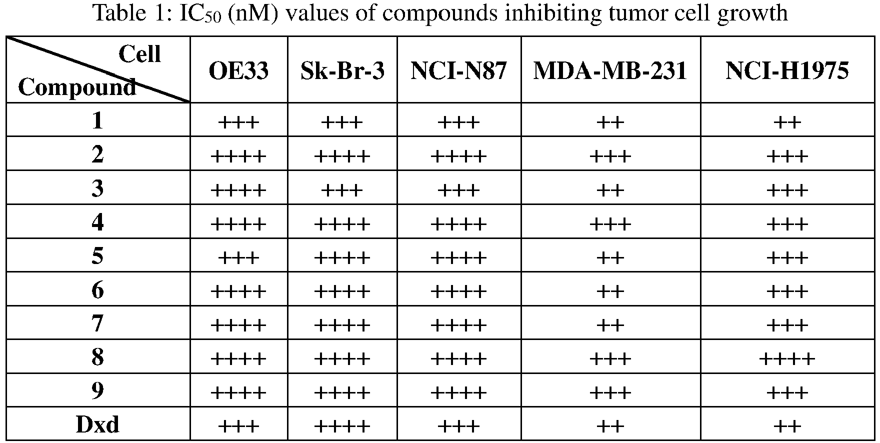

All the compounds provided in examples of the present disclosure have good inhibitory function against the growth of cancer cells, and have significant anticancer activity.

The foregoing description of specific exemplary embodiments of the present disclosure is provided for purposes of illustration and exemplification. These descriptions are not intended to limit the present disclosure to the precise forms disclosed; rather, it is evident that, in light of the foregoing teachings, numerous modifications and variations are possible. The selection and description of these exemplary embodiments are intended to explain the specific principles of the present disclosure and their practical applications, thereby enabling those skilled in the art to implement and utilize various exemplary embodiments of the present disclosure, as well as various selections and modifications. The scope of the present disclosure is intended to be defined by the appended claims and their equivalents.

## Claims

1. A compound of Formula (I), and a stereoisomer or a pharmaceutically acceptable salt thereof: 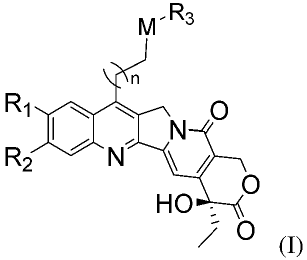
in the formula,
R₁ and R₂ are each independently selected from hydrogen, fluorine, and C₁₋₃ alkyl; or R₁ and R₂ together with the carbon atoms to which they are attached form an oxygen-containing heterocyclyl;
M is selected from -NH-O- and -O-NH-;
n is selected from 0, 1, 2, and 3; and
R₃ is selected from hydrogen, C₁₋₃ alkyl, or C₃₋₆ cycloalkyl.

2. The compound according to claim 1, wherein R₁ and R₂ are each independently selected from hydrogen, fluorine, and methyl, or R₁ and R₂ together with the carbon atoms to which they are attached form 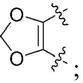
preferably, R₁ is hydrogen, and R₂ is hydrogen;
R₁ is fluorine, and R₂ is fluorine;
R₁ is methyl, and R₂ is fluorine; or
R₁ and R₂ together with the carbon atoms to which they are attached form

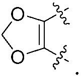

3. The compound according to claim 1 or 2, wherein R₃ is selected from C₁₋₃ alkyl, more preferably methyl.

4. The compound according to any one of claims 1-3, wherein the compound of Formula (I) has a structure as shown in Formula (IA) below: 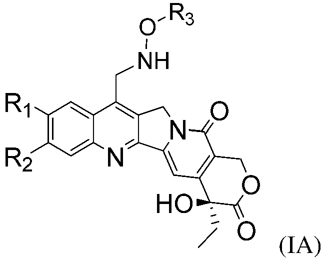
in the formula, R₁, R₂ and R₃ are each the same as defined for the compound of Formula (I);
preferably, R₁ is hydrogen, and R₂ is hydrogen;
R₁ is fluorine, and R₂ is fluorine;
R₁ is methyl, and R₂ is fluorine; or
R₁ and R₂ together with the carbon atoms to which they are attached form 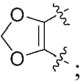 and
R₃ is methyl.

5. The compound according to any one of claims 1-3, wherein the compound of Formula (I) has a structure as shown in Formula (IB) below: 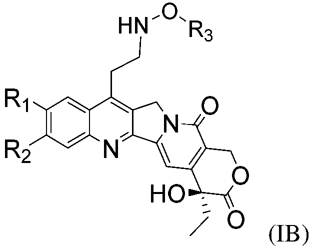
in the formula, R₁, R₂ and R₃ are each the same as defined for the compound of Formula (I);
preferably, R₁ is hydrogen, and R₂ is hydrogen;
R₁ is fluorine, and R₂ is fluorine;
R₁ is methyl, and R₂ is fluorine; or
R₁ and R₂ together with the carbon atoms to which they are attached form 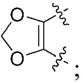 and
R₃ is methyl.

6. The compound according to any one of claims 1-3, wherein the compound of Formula (I) has a structure as shown in Formula (IC) below: 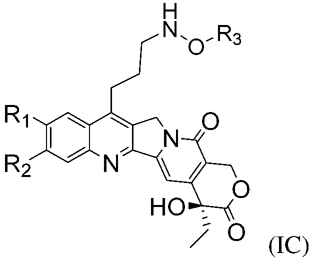
in the formula, R₁, R₂ and R₃ are each the same as defined for the compound of Formula (I);
preferably, R₁ is hydrogen, and R₂ is hydrogen;
R₁ is fluorine, and R₂ is fluorine;
R₁ is methyl, and R₂ is fluorine; or
R₁ and R₂ together with the carbon atoms to which they are attached form 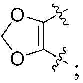 and
R₃ is methyl.

7. The compound as described below, and a stereoisomer or a pharmaceutically acceptable salt thereof:

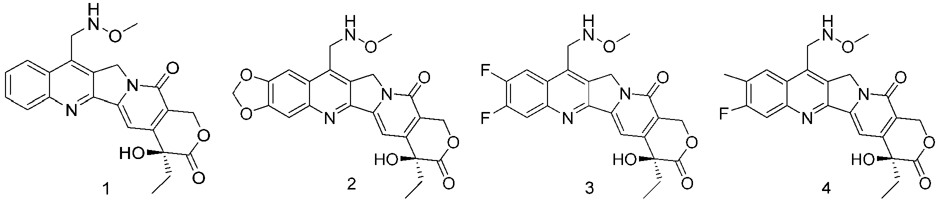

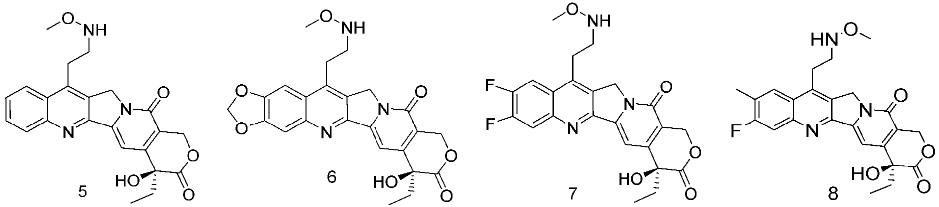

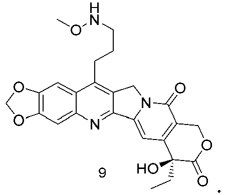

8. A preparation method of the compound according to any one of the claims 1-7, which is selected from following synthetic routes:
synthetic route 1: 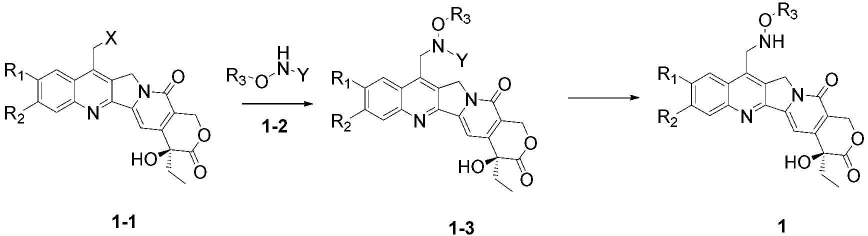
a compound of Formula (1-1) and a compound of Formula (1-2) are subjected to a substitution reaction to obtain a compound of Formula (1-3);
Y group is removed from the compound of Formula (1-3) to obtain the compound of Formula (1);
synthetic route 2: 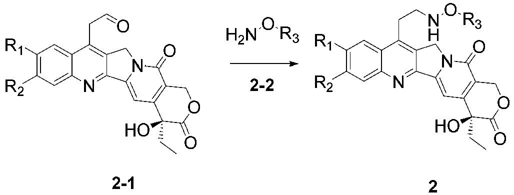
a compound of Formula (2-1) and a compound of Formula (2-2) are subjected to a reductive amination reaction to obtain a compound of Formula (2);
in the formula, R₁, R₂, and R₃ are each the same as defined for the compound of Formula (I);
X is selected from halogen, preferably bromine; and
Y is selected from amino protecting groups, preferably p-methoxybenzyl.

9. A pharmaceutical composition, comprising the compound, the stereoisomer or the pharmaceutically acceptable salt thereof according to any one of claims 1-7; and a pharmaceutically acceptable carrier.

10. Use of the compound, the stereoisomer or the pharmaceutically acceptable salt thereof according to any one of claims 1-7, or the pharmaceutical composition according to claim 9 in the manufacture of an antitumor drug;
preferably, the tumor is selected from a solid tumor, more preferably selected from one or more of breast cancer, esophageal cancer, gastric cancer, and lung cancer.

11. A method for treating cancer, comprising a step of administering the compound, the stereoisomer or the pharmaceutically acceptable salt thereof according to any one of claims 1-7, or the pharmaceutical composition according to claim 9 to a patient in need thereof;
preferably, the cancer is selected from one or more of breast cancer, esophageal cancer, gastric cancer, and lung cancer.
